# EUROPÄISCHE PATENTANMELDUNG

(11) **EP 1 403 234 A2**
(43) Veröffentlichungstag der Anmeldung: **31.03.2004**
(21) Anmeldenummer: 03019797.4
(22) Anmeldetag: 30.08.2003
(51) Int. Cl.: C07B 33/00, C07C 409/04

(54) **Verfahren zur Singlet Sauerstoff Oxidation von organischen Substraten**

(30) Priorität: 26.09.2002 AT 14432002
(71) Anmelder: DSM Fine Chemicals Austria Nfg GmbH & Co KG, 4021 Linz (AT)
(72) Erfinder: Emsenhuber, Martin, 4040 Linz (AT); Kwant, Gerard, 6137 LM Sittard (NL); van Straaten, Koenraad, 6114 MG Echt-Susteren (NL); Janssen, Madelon, 3500 Hasselt (BE); Alsters, Paul, 6224 KZ Maastricht (NL); Hoving, Hendrik, 6214 PC Maastricht (NL)
(74) Vertreter: Lindinger, Ingrid

(57) **Zusammenfassung**

Verbessertes Verfahren zur Oxidation von organischen Substraten mittels ¹O₂, bei welchem wasserlösliche, oder in mit Wasser mischbaren organischen Lösungsmitteln lösliche, organische Substrate, die mit ¹O₂ reagieren, in einem mit Wasser mischbaren organischen Lösungsmittel, in Wasser oder in einem Gemisch aus Wasser und mit Wasser mischbaren organischem Lösungsmittel in Gegenwart eines heterogenen oder homogenen Katalysators mit 3-90%igem H₂O₂ versetzt werden, worauf anschließend an die katalytische Zersetzung von H₂O₂ zu Wasser und ¹O₂ die Oxidation der Substrate zu den entsprechenden Oxidationsprodukten erfolgt, wobei während der Reaktion Wasser selektiv mittels Membranen aus dem Reaktionsgemisch entfernt wird.

## Beschreibung

Die Erfindung betrifft ein verbessertes Verfahren zur Singlet Sauerstoff Oxidation von organischen Substraten, bei welchem mittels Membranen während der Reaktion selektiv Wasser aus dem Reaktionsgemisch entfernt wird.

Die einzige Singlet Sauerstoff Oxidation (¹O₂-Oxidation), die zur Zeit industriell durchgeführt wird, ist die photochemische ¹O₂-Oxidation, bei welcher der ¹O₂ auf photochemischen Weg generiert wird. Der Nachteil dieses Verfahrens ist durch die hohen Kosten der benötigten photochemischen Einrichtungen, sowie durch eine beschränkte Lebensdauer derselben gegeben. Die benötigten Lampen degenerieren durch Verschmutzung der Glasoberfläche relativ rasch während der Oxidation. Außerdem eignet sich dieses Verfahren nicht für gefärbte Substrate. Das Verfahren ist eigentlich nur für Feinchemikalien geeignet, die im kleineren Maßstab hergestellt werden. (La Chimica e l'Industria, 1982, Vol. 64, Seite 156)
Aus diesem Grund wurde versucht, andere Verfahrensvarianten für die ¹O₂-Oxidation zu finden, bei welchen ¹O₂ chemisch statt photochemisch generiert wird.

In J. Am. Chem. Soc., 1968, **90**, 975 wird beispielsweise die klassische "dark" ¹O₂-Oxidation beschrieben, bei welcher ¹O₂ nicht photochemisch, sondern chemisch generiert wird. Dabei werden hydrophobe Substrate mittels eines Hypochlorit/H₂O₂ - Systems in einem Lösungsmittelgemisch aus Wasser und organischem Lösungsmittel oxidiert. Die Einsatzmöglichkeit dieses Verfahrens ist aufgrund von Nebenreaktionen zwischen Hypochlorit und Substrat oder Lösungsmittel ziemlich eingeschränkt. Weiters ist dieses Verfahren nicht für den industriellen Maßstab geeignet, da es im organischen Medium zur Anlagerung des Hypochlorits an H₂O₂ kommt und ein großer Überschuss an H₂O₂ zur Unterdrückung der Nebenreaktion von Substrat mit Hypochlorit benötigt wird. Ein zusätzlicher Nachteil ergibt sich durch das Anfallen stöchiometrischer Salzmengen.

Eine Variante der "dark" ¹O₂-Oxidation, die nicht auf Hypochlorit basiert und somit obige Nachteile zum Teil vermeiden soll, ist beispielsweise aus J. Org. Chem., 1989, **54**, 726 oder J. Mol. Cat., 1997, **117**, 439 bekannt, wonach einige wasserlösliche organische Substrate mit H₂O₂ und einem Molybdatkatalysator in Wasser als Lösungsmittel oxidiert werden.

Der Nachteil der bisher bekannten "dark" ¹O₂-Oxidation, die in Wasser oder einem Gemisch aus Wasser und organischem Lösungsmittel durchgeführt werden, ist jedoch, wie beispielsweise in J. Am. Chem. Soc. **1997**, 119, S. 5286 beschrieben, dass der Großteil des ¹O₂ durch Quenching durch die Wassermoleküle verloren geht, sodass ein großer Überschuss an H₂O₂ benötigt wird, um annehmbare Umsätze zu erzielen.

Da üblicherweise H₂O₂ als bis zu 30 oder 35%ige wässrige Lösung erhältlich ist und zudem bei der Bildung von ¹O₂ auch Wasser entsteht, leiden Reaktionen, bei denen der als Oxidationsmittel verwendete Singletsauerstoff in situ aus H₂O₂ gewonnen wird, zudem unter der zusätzlichen Verdünnung des Reaktionsmediums mit Wasser.
Dies führt auch dazu, dass bei Batch-Reaktionen Reaktionsvolumen verloren geht, wodurch die Raum-Zeit-Ausbeute ebenfalls verringert wird.

Ein weiterer Nachteil von "dark" ¹O₂-Oxidation in Wasser oder in Wasser/Lösungsmittelgemischen besteht darin, dass bei manchen organischen Substraten, insbesondere bei solchen mit höherem Molekulargewicht, bei einem hohen Wassergehalt des Reaktionsmediums eine Entmischung bzw. eine Phasentrennung auftritt, die sich sehr negativ auf die Ausbeute auswirkt.

Aufgabe der vorliegenden Erfindung war es demnach, eine verbesserte Methode der "dark" ¹O₂-Oxidation zu finden, die einfach, kostengünstig und umweltfreundlich im industriellen Maßstab angewendet werden kann, wobei die Nachteile, die durch die Anwesenheit von Wasser entstehen, beseitigt werden.

Unerwarteterweise wurde nun gefunden, dass die "dark" ¹O₂-Oxidation in äußerst effizienter Weise mit hoher Ausbeute in Wasser oder in Wasser/Lösungsmittelgemischen durchgeführt werden kann, wenn Wasser während der Reaktion selektiv mittels Membranen aus dem Reaktionsgemisch entfernt wird.

Gegenstand der vorliegenden Erfindung ist demnach ein verbessertes Verfahren zur Oxidation von organischen Substraten mittels ¹O₂, das dadurch gekennzeichnet ist, dass wasserlösliche, oder in mit Wasser mischbaren organischen Lösungsmitteln lösliche, organische Substrate, die mit ¹O₂ reagieren, in einem mit Wasser mischbaren organischen Lösungsmittel, in Wasser oder in einem Gemisch aus Wasser und mit Wasser mischbaren organischem Lösungsmittel in Gegenwart eines heterogenen oder homogenen Katalysators mit 3-90%igem H₂O₂ versetzt werden, worauf anschließend an die katalytische Zersetzung von H₂O₂ zu Wasser und ¹O₂ die Oxidation der Substrate zu den entsprechenden Oxidationsprodukten erfolgt, wobei während der Reaktion Wasser selektiv mittels Membranen aus dem Reaktionsgemisch entfernt wird.

Das erfindungsgemäße Verfahren eignet sich zur Oxidation von wasserlöslichen, oder in mit Wasser mischbaren organischen Lösungsmitteln löslichen, organischen Substraten, die mit ¹O₂ reagieren.
Geeignete Substrate sind beispielsweise in WO 00/64842 und WO 00/61524 beschrieben. Ausgenommen sind solche Substrate, die hoch hydrophob sind, wie etwa Rubren, und solche die in Wasser oder in einem mit Wasser mischbaren organischen Lösungsmittel nicht löslich sind.
Als Substrat können demnach folgende Verbindungen eingesetzt werden: Olefine, die eine oder mehrere, d.h. bis zu 10, bevorzugt bis zu 6, besonders bevorzugt bis zu 4 C=C-Doppelbindungen enthalten; elektronenreiche Aromaten, wie C₆-C₃₀₋, bevorzugt bis C₂₀₋, besonders bevorzugt bis C₁₅-Aromaten, wie etwa Phenole, Polyalkylbenzole, Polyalkoxybenzolen u.s.w.; polycyclische Aromaten mit 2 bis 8, bevorzugt bis 4, besonders bevorzugt bis 3 aromatischen Ringen; Sulfide, wie etwa Alkylsulfide, Alkenylsulfide, Arylsulfide, die am Schwefelatom entweder mono- oder disubstituiert sind, sowie Heterocyclen mit einem oder mehreren O-, N- oder S-Atomen im Ring, wie beispielsweise C₄-C₃₀₋, bevorzugt bis C₂₀₋, besonders bevorzugt bis C₁₅ - Heterocyclen, wie etwa Furane, Pyrrole, Thiophene, Thiazole, Pyridine, Isobenzofurane, Chinoline u.s.w..
Die Substrate können dabei einen oder mehrere Substituenten, wie Halogen (F, Cl, Br, J), Cyanid, Carbonylgruppen, Hydroxylgruppen, C₁-C₂₀, bevorzugt bis C₁₀, besonders bevorzugt bis C₆- Alkoxygruppen, C₁-C₂₀-, bevorzugt bis C₁₀-, besonders bevorzugt bis C₆-Alkylgruppen, C₆-C₃₀-, bevorzugt bis C₂₀, besonders bevorzugt bis C₁₀-Arylgruppen, C₂-C₂₀, bevorzugt bis C₁₀, besonders bevorzugt bis C₆-Alkenylgruppen, C₂-C₂₀, bevorzugt bis C₁₀, besonders bevorzugt bis C₆-Alkinylgruppen, Carbonsäuregruppen, Estergruppen, Amidgruppen, Aminogruppen, Nitrogruppen, Silylgruppen, Silyloxygruppen, Sulfongruppen, Sulfoxidgruppen, aufweisen.
Weiters können die Substrate substituiert sein mit einem oder mehreren NR¹ R²-Resten, in denen R₁ oder R₂ gleich oder verschieden sein können und H; C₁-C₂₀, bevorzugt bis C₁₀, besonders bevorzugt bis C₆- Alkyl; Formyl; C₂-C₂₀, bevorzugt bis C₁₀, besonders bevorzugt bis C₆- Acyl; C₇-C₃₀, bevorzugt bis C₂₀, besonders bevorzugt bis C₁₀ -Benzoyl bedeuten, wobei R¹ und R² auch gemeinsam einen Ring bilden können, wie z.B. in einer Phthalimidogruppe.

Beispiele für besonders geeignete Substrate sind: 1,3-Butadien; 2,3-Dimethylbuten; D^{9,10}-Octalin, 2,3-Dimethyl-1,3-Butadien; 2,4-Hexadien; 1,3-Cyclohexadiene, 4-Methyl-3-penten-2-ol, 3,4-Dimethyl-3-penten-2-ol, 3-(4-Methyl-1-naphthyl)-propionsäure, 3,3'-(Naphthalen-1,4-diyl)-dipropionsäure, 1-Trimethylsilylcyclohexen; (E)-2-Methylcrotonsäure, 2,3-Dimethyl-2-butenyl-*para*-tolylsulfon; 2,3-Dimethyl-2-butenyl-*para*-tolylsulfoxid; *N*-Cyclohexenylmorpholin; 2-Methyl-2-norbornen; Terpinolen; α-Pinen; β-Pinen; β-Citronellol; Ocimen; Citronellol; Geraniol; Farnesol; Terpinen; Limonen; trans-2,3-Dimethylacrylsäure; α-Terpinen; Isopren; Cyclopentadien; 1,4-Diphenylbutadien; 2-Ethoxybutadien; 1,1'-Dicyclohexenyl; Cholesterol; Ergosterolacetat; 5-Chlor-1,3-cyclohexadien; 3-Methyl-2-buten-1-ol; 2-Phthalimido-4-methyl-3-penten-2-ol, Phenol, 1,2,4-Trimethoxybenzol, 2,3,6-Trimethylphenol, 2,4,6-Trimethylphenol, 1,4-Dimethylnaphthalen, Furan, Furfurylalkohol, Furfural, 2,5-Dimethylfuran, Isobenzofuran, Dibenzylsulfid, (2-Methyl-5-*tert*-butyl)phenylsulfid, Dibenzylsulfid, Diphenylsulfid, u.s.w.

Die Substrate können auch in Form eines Salzes, beispielsweise als Na- oder K-Salz oder als Tetra-C₁-C₆- alkylammoniumsalz eingesetzt werden.

Aus den Substraten wird durch die erfindungsgemäße Oxidation das korrespondierende Oxidationsprodukt erhalten. Aus Alkenen, (polycyclischen) Aromaten oder Heteroaromaten werden insbesondere Hydroperoxide oder Peroxide erhalten, die unter den Reaktionsbedingungen zu Alkoholen, Epoxiden, Acetalen oder Carbonylverbindungen, wie Ketone, Aldehyde, Carbonsäuren oder Ester weiter reagieren können, wenn das Hydroperoxid oder das Peroxid nicht stabil ist.

Die erfindungsgemäße Oxidation erfolgt in einem mit Wasser mischbaren organischen Lösungsmittel oder in Wasser oder in einem Gemisch aus Wasser und einem mit Wasser mischbaren organischem Lösungsmittel.
Geeignete mit Wasser mischbare Lösungsmittel sind C₁-C₈-Alkohole, wie Methanol, Ethanol, Propanol, i-Propanol, Butanol, i-Butanol; Ethylenglykol, Propylenglykol, Formamid, N-Methylformamid, Dimethylformamid (DMF), Sulfolan, Dioxan, THF und 1,2-Dimethoxyethan.
Bevorzugt werden Methanol, Ethanol, Propanol, i-Propanol, Dioxan, DMF und THF, besonders bevorzugt Methanol, Ethanol oder THF als mit Wasser mischbare Lösungsmittel eingesetzt.

Dem Lösungsmittel-Substrat-Gemisch wird als heterogener oder homogener anorganischer Katalysator ein Metall, das sich für ¹O₂-Oxidationidationen eignet und beispielsweise in WO 00/64842 und WO 00/61524, J. Am. Chem. Soc., 1985, **107**, 5844, Chem. Eur. J. (2001) 7(12), S.2547-2556 oder in Membrane Lipid Oxid. Vol. II, 1991, 65 beschrieben ist, zugesetzt.

Bevorzugt werden Katalysatoren basierend auf Molybdän, Wolfram, Scandium, Vanadium, Titan, Zirkon, Praseodym, Neodym, Samarium, Europium, Terbium, Dysprosium, Holmium, Erbium, Ytterbium, Lanthan, Cer oder Gadolinium, sowie die sogenannten Pseudo-Lanthaniden Scandium und Yttrium und Lutetium eingesetzt. Besonders bevorzugt sind Molybdän- und Lanthan-Katalysatoren.

Das Metall kann dabei in für ¹O₂-Oxidationidationen üblichen Formen, beispielsweise als Oxid, Oxokomplex, Nitrat, Carboxylat, Hydroxid, Layered Double Hydroxid (LDH), Carbonat, Chlorid, Fluorid, Sulfat, Tetrafluorborat, u.s.w., vorliegen. Homogenen, löslichen Formen des Katalysators kann gegebenenfalls ein Hydroxid, beispielsweise NaOH, KOH, u.s.w., zugegeben werden, sodass ein heterogener, aktiver Katalysator erhalten wird.

Die Menge an eingesetztem Katalysator hängt vom eingesetzten Substrat ab und liegt zwischen 1 und 50 mol%, bevorzugt zwischen 5 und 25 mol% Gegebenenfalls kann es zur besseren Aktivierung des Katalysators von Vorteil sein, wenn dem Reaktionsgemisch übliche basische oder saure Zusätzen zugegeben werden.

Anschließend erfolgt die Zugabe von 3-90%igem, bevorzugt 30-60%igem H₂O₂. Bevorzugt wird H₂O₂ langsam oder portionsweise dem Reaktionsgemisch aus Lösungsmittel, Substrat und Katalysator zugegeben, wobei das Reaktionsgemisch gerührt wird. Es ist auch möglich zuerst nur einen Teil des H₂O₂, anschließend ein Hydroxid, wie etwa NaOH, KOH u.s.w., und danach die restliche Menge an H₂O₂ zuzugeben.

Der Verbrauch an H₂O₂ ist bei dem erfindungsgemäßen Verfahren vom eingesetzten Substrat abhängig. Für reaktive Substrate werden bevorzugt 2 bis 3 Äquivalente an H₂O₂ benötigt, während weniger reaktive Substrate bevorzugt mit 3 bis 10 Äquivalenten an H₂O₂ umgesetzt werden.

Die Reaktionstemperatur liegt zwischen 0 und 50°C, bevorzugt zwischen 15 und 35°C.

Der pH-Wert des Reaktionsgemisches hängt von dem gewählten Substrat und dem gewählten Katalysator ab und kann dabei zwischen 0 und 14, bevorzugt zwischen 4 und 14 liegen. Der pH-Wert des Reaktionsgemisches kann dabei, wenn notwendig, je nach Bedarf mit üblichen basischen oder sauren Zusätzen eingestellt werden.

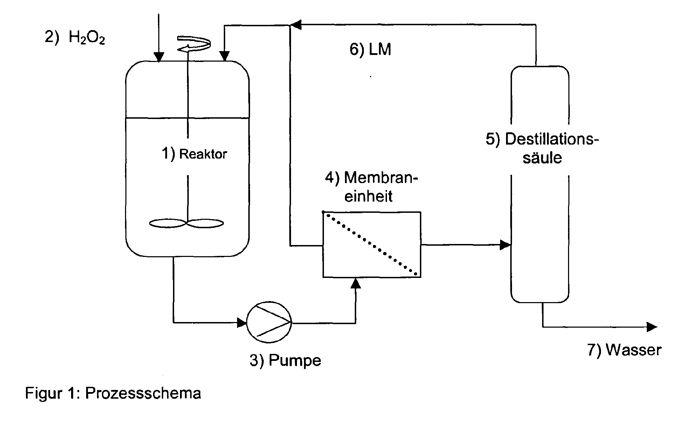

Erfindungsgemäß wird bei dem vorliegenden Verfahren während der Reaktion selektiv Wasser mittels Membranen entfernt.
Dabei wird Wasser, das gegebenenfalls als Lösungsmittel eingesetzt wird und Wasser, das durch die 2-90%ige H₂O₂-Lösung eingebracht und das bei der katalysierten Disproportionierung von H₂O₂ gebildet wird, und gegebenenfalls vorhandenes mit Wasser mischbares organisches Lösungsmittel durch eine Membraneinheit simultan aus dem Reaktionsgemisch entfernt, worauf anschließend gegebenenfalls eine destillative Abtrennung des Wassers vom organischen Lösungsmittel erfolgt. Das organische Lösungsmittel wird sodann wieder in den Reaktor eingebracht.
Das Prozessschema ist aus Figur 1 ersichtlich und zeigt einen Reaktor (1), in dem das Lösungsmittel bzw. Lösungsmittelgemisch, das Substrat und der Katalysator vorgelegt werden und in den sodann H₂O₂ durch Leitung (2) eingebracht wird. Über eine Pumpe (3) gelangt das Reaktionsgemisch in die Membraneinheit (4). In der Membraneinheit (4) wird sodann Wasser (Permeat) durch eine geeignete Membran abgetrennt, wobei der Katalysator (im Falle eines homogenen Katalysators), das noch nicht umgesetzte Substrat und bereits gebildetes Produkt zurückgehalten werden (Retentat) und sogleich wieder in den Reaktor eingeleitet werden.
In Abhängigkeit von der gewählten Membran wird das gegebenenfalls verwendete mit Wasser mischbare organische Lösungsmittel entweder ebenfalls zurückgehalten, wie das beispielsweise für MeOH bei Verwendung von anorganischen Membranen der Fall ist, oder aber mit dem Wasser abgetrennt.
Für den Fall, dass das organische Lösungsmittel mit dem Wasser vom Reaktionsgemisch abgetrennt wird, erfolgt sodann eine destillative Auftrennung (Destillationssäule (5)) von Wasser und mit Wasser mischbaren Lösungsmittel, worauf das Lösungsmittel wieder dem Reaktor zugeführt wird (Leitung (6)) und das abgetrennte Wasser verworfen wird.

Bei dem erfindungsgemäßen Verfahren können anstelle einer Membraneinheit auch zwei oder mehr Membraneinheiten eingesetzt werden. Bei Verwendung von zwei Membraneinheiten wird dabei das Permeat der ersten Einheit in die zweite Membraneinheit geleitet und das Retentat der ersten Membraneinheit wieder, wie oben beschrieben, in den Reaktor rückgeführt. Anschließend wird das Retentat der zweiten Einheit ebenfalls wieder in den Reaktor geleitet, während das Permeat der zweiten Einheit wie oben beschrieben aufgearbeitet (Abdestillieren von Lösungsmittel) oder, wenn das Permeat nur Wasser enthält, verworfen wird.

Für das erfindungsgemäße Verfahren eignen sich sowohl organische als auch anorganische Membranen.

Als organische Membranen eignen sich Membranen für die umgekehrte Osmose (reversed osmosis R.O.), die beispielsweise aus Polyvinylalkohol, Polyamid oder Polysulfon oder Celluloseacetat, Cellulosediacetat, Cellulosetriacetat, Cellulosediacetat/Cellulosetriacetat (CDA/CTA), Cellulosenitrate, Polypropylen, Polyimid, sulfonated polysulfones, Polyethersulfone, Polyacrylnitril, Polyimid/Polyetherimid, Polyvinylidenfluorid, Aramid oder Polypiperazin oder Gemische derselben bestehen.
Bevorzugt sind solche Membrane, die Moleküle mit einem Molekulargewicht zwischen 50 und 200 Da (g/mol), besonders bevorzugt zwischen 50 und 100Da zurückhalten.

Weiters eignen sich als organische Membranen Membranen für die Nanofiltration, deren aktive Separationsschicht beispielsweise Polyvinylalkohol, Polypropylen, Polysulfon, Polyethersulfon, Polyacrylnitril, Polyimid/Polyetherimid, Polyvinylidenfluorid, Polyamid, Polypiperazin, Celluloseacetat, Cellulosenitrat, Aramid, Cellulosediacetat, Cellulosetriacetat, u.s.w. oder Gemische derselben, enthält.
Bevorzugt sind solche Nanofiltrationsmembranen, die Moleküle mit einem Molekulargewicht zwischen 50 und 400 Da (g/mol), besonders bevorzugt zwischen 50 und 200Da zurückhalten.

Bevorzugt werden als organische Membranen R.O.Membranen eingesetzt, die eigentlich für alle suspendierten und gelösten Materialien geeignet sind, da diese alle zurückgehalten werden.

Im Handel erhältliche R.O.Membranen sind beispielsweise Toray SU810 (Fa. Toray, auf Basis von Polyamid), PCI ACF99 (Fa. PCI, auf Basis von Polyamid), DESAL SC (Fa. Osmonics-Desalination Systems, auf Basis von Celluloseacetat), Pall Rochem 05757, SW 30 HR(Fa Film Tec-DOW, auf Basis von Polyamid), Trisep X-20(Fa. Trisep, auf Basis von ACM/Celluloseacetat), NTR 759(Fa. Nitto Denko; auf Basis von Polyvinylalkohol/polyamid), NTR 729(Fa. Nitto Denko; auf Basis von Polyvinylalkohol); Pervap1510 (Fa. Deutsche Carbone AG), CMC-CE01 (Fa. Celfa, auf Basis von Polyvinylalkohol) u.s.w.

Im Handel erhältliche N.F.-Membranen sind beispielsweise Desal 5K (Fa. Osmonics-Desalination Systems; auf Basis von Polyamid) Koch MPF60 (Koch-Membrane Products), NF200 (Filmtec DOW; auf Basis von Polypiperazinamid), NF CA30 (Fa. Nadir; auf Basis Celluloseacetat) u.s.w.

Als organische Membranen kommen zudem auch organische Pervaporationsmembranen in Frage, deren aktive Separationsschicht Polydimethylsiloxane, Poly(1-trimethylsilyl-1-propin), Polyurethane, Polybutadien, Polyether-Block-Polyamide, Silicon-Polycarbonate, Styrol-Butadien-Kautschuk, Nitril-Butadien-Kautschuk, Ethen-Propen-Terpolymer, Polyvinylalkohol, Polyamid, Polysulfon, Celluloseacetat, Aramid, Cellulosediacetat, Cellulosetriacetat, Polypiperazin u.s.w oder Gemische derselben, enthält.
Organische Pervaporationsmembranen zeichnen sich dadurch aus, dass sie Wasser effektiv von Molekülen, die größer als 4.5 Å sind, abtrennen. Bevorzugt werden sie zur Abtrennung von Molekülen in einer Größe über 5 Å eingesetzt.

Als anorganische bzw. keramische Membranen werden bevorzugt Membranen für die Pervaporationtechnik eingesetzt. Dies sind Membranen, die beispielsweise aus einer aktiven Schicht auf Basis von Aluminium, Titanoxid, Silica, Boroxid, Magnesium, Zirkonium, Ton u.s.w. oder Gemischen derselben, auf einem geeigneten Träger, wie etwa einem gamma-Al₂O₃ Träger, bestehen.
Im Handel erhältliche Pervaporationsmembranen sind beispielsweise Sulzer Pervap SMS (Fa. Sulzer, Silica Membransystem)

Auch diese anorganischen Pervaporationsmembranen zeichnen sich dadurch aus, dass sie Wasser effektiv von Molekülen, die größer als 4.5 Å sind, abtrennen. Bevorzugt werden sie zur Abtrennung von Molekülen in einer Größe über 5 Å eingesetzt.

Weiters eignen sich keramische Nanofiltrationsmembranen auf Basis von Aluminium, Boroxid, Magnesium, Zirkon, TiO₂, SiO₂, Ton u.s.w oder Gemischen derselben, beispielsweise von HITK (Hermsdorfer Institut für Technische Keramik), und TAMI (Tami Industries) u.s.w., sowie Zeolithmembranen auf Basis von Aluminium, Boroxid, Magnesium, Zirkon, TiO₂, SiO₂, Ton u.s.w oder Gemischen derselben.

Bevorzugt werden für das erfindungsgemäße Verfahren keramische Membranen eingesetzt.

Die wichtigsten Parameter für geeignete Membranen sind dabei:
- Kapazität: Permeatfluss, kg/m².hr.bar
- Selektivität: Retentions- oder Separationsfaktor,
- mechanische Stabilität: Abrieb, Temperatur
- chemische Stabilität: gegenüber Lösungsmittel und Oxidationsmittel

Geeignete Membranen sollten einen Retentionsfaktor für Substrat und Produkt von mindestens 85%, bevorzugt von mindestens 95% aufweisen, um Substrat- und Produktverluste möglichst gering zu halten.
Werden zwei Membraneinheiten verwendet, so können Membranen mit einem geringeren Retentionsfaktor von mindestens 80%, bevorzugt von mindestens 90% eingesetzt werden.

Die Auswahl einer geeigneten Membran erfolgt dabei in Abhängigkeit vom gewählten Reaktionsmedium, Substrat, Produkt und Katalysator.

Bevorzugt werden geeignete Membranen in Vorversuchen, in denen die Stabilität im Reaktionsmedium, der Retentionsfaktor bezüglich Substrat und Produkt, die Kapazität (Permeatfluss) u.s.w. untersucht wird, ermittelt.

Durch das erfindungsgemäße Verfahren wird vermieden, dass das Reaktionsgemisch zunehmend durch das mit dem Wasserstoffperoxid (durch Einsatz einer 3-90%igen Lösung und durch Disproportionierung in Wasser und ¹O₂) eingebrachte Wasser verdünnt wird. Dadurch werden Ausbeuteverluste bei der Batchfahrweise und negative Einflüsse auf die Löslichkeit (Entmischung u.s.w.) und die Effizienz des ¹O₂ verhindert. Weiters werden negative Einflüsse von Wasser auf die Stereoselektivität der Reaktion verhindert.
Durch die erfindungsgemäße Wasserabtrennung können demnach die Ausbeuten bei der Batchfahrweise um bis zu über 200% gesteigert werden und die Stereoselektivität der Reaktion erhöht werden.

### Vergleichsversuch: ohne Wasserentfernung

In einem Reaktor wurde ein Gemisch aus Methanol, 2-Methylcrotonsäure und Na₂MoO₄-Katalysator vorgelegt. Anschließend wurde eine 30 Gew.%ige H₂O₂-Lösung zugegeben. Das Batchvolumen erhöhte sich dabei durch das Wasser, das sich bei der katalysierten Disproportionierung von H₂O₂ bildete, und um das Volumen an Wasser der 30 Gew.%ige H₂O₂-Lösung. Das Gesamtvolumen am Ende des Batchversuches bestimmt die maximale Anfangsbeladung des Reaktors bzw. die Batch-ausbeute bei Beendigung der Reaktion.

Aus der Tabelle 1 sind die Reaktionsparameter ersichtlich

| H₂O₂/Substrat, mol/mol | Water -% Batchende Batchende Gew.% | [Substrat] mol/l | Batchausbeute kg |
|---|---|---|---|
| 2 | 5.0 | 0.22 | 196 |
| 2 | 30.0 | 1.93 | 1333 |
| 2 | 18.9 | 1 | 795 |
| 2 | 49.5 | 5.1 | 2431 |
| 10 | 5.0 | 0.04 | 39 |
| 10 | 30.0 | 0.36 | 251 |
| 10 | 53.9 | 1 | 481 |
| 10 | 84.9 | 5 | 810 |

### Beispiel 1: Wasserentfernung mit einer Membraneinheit

In einem Reaktor wurde ein Gemisch aus Methanol, Substrat und Na₂MoO₄-Katalysator vorgelegt. Anschließend wurde eine 30 Gew.%ige H₂O₂-Lösung zugegeben. Das Batchvolumen erhöhte sich dabei durch das Wasser, das sich bei der katalysierten Disproportionierung von H₂O₂ bildete, und um das Volumen an Wasser der 30 Gew.%ige H₂O₂-Lösung.

Nachdem der vorher bestimmte Gesamtgehalt an Wasser erreicht wurde, wurde die Membraneinheit (Firma Celfa) in Betrieb genommen, um weiteres zugegebene und sich bildende Wasser zu entfernen. Ebenfalls entferntes Methanol wurde vollständig durch destillative Auftrennung wieder gewonnen und in den Reaktor rückgeführt.

**Tabelle 2:**

| Batchausbeute für ein leicht oxidierbares Substrat wie etwa β-Citronellol mit einem H₂O₂/Substrat -Molverhältnis = 2; | | | | |
|---|---|---|---|---|
| Retention | Wassergehalt Gew.% | Batchausbeute kg | %-Erhöhung gegenüber Vgl.bsp. | Ausbeute % |
| 90 | 5 | 609 | 211 | 64 |
| 95 | 5 | 759 | 287 | 80 |
| 99 | 5 | 908 | 363 | 96 |
| 100 | 5 | 950 | 385 | 100 |

**Tabelle 3:**

| Batchausbeute für ein schwer oxidierbares Substrat, wie etwa Tiglic acid mit einem H₂O₂/Substrat -Molverhältnis = 10; | | | | |
|---|---|---|---|---|
| Retention | Wassergehalt Gew.% | Batchausbeute kg | %-Erhöhung gegenüber Vgl.bsp. | Ausbeute % |
| 90 | 5 | 91 | 133 | 10 |
| 95 | 5 | 291 | 646 | 31 |
| 99 | 5 | 748 | 1818 | 78 |
| 100 | 5 | 950 | 2336 | 100 |
| 90 | 30 | 677 | 170 | 71 |
| 95 | 30 | 801 | 219 | 84 |
| 99 | 30 | 917.8 | 266 | 96.6 |
| 100 | 30 | 950 | 278 | 100 |

### Beispiel 2: Wasserentfernung mit zwei Membraneinheiten

Der Versuch wurde analog Beispiel 1 durchgeführt, es wurden jedoch zur Wasserentfernung zwei Membraneinheiten verwendet.

**Tabelle 4:**

| Batchausbeute für ein leicht oxidierbares Substrat mit einem H₂O₂/Substrat -Molverhältnis = 2; | | | | |
|---|---|---|---|---|
| Retention | Wassergehalt Gew.% | Batchausbeute kg | Erhöhung gegenüber Vgl.bsp. | Ausbeute % |
| 90 | 5 | 898 | 358 | 95 |
| 95 | 5 | 937 | 378 | 98.6 |
| 99 | 5 | 949.5 | 384 | 99.94 |
| 100 | 5 | 950 | 385 | 100 |

**Tabelle 5:**

| Batchausbeute für ein schwer oxidierbares Substrat mit einem H₂O₂/Substrat -Molverhältnis = 10; | | | | |
|---|---|---|---|---|
| Retention | Wassergehalt Gew.% | Batchausbeute kg | %-Erhöhung gegenüber Vgl.bsp. | Ausbeute % |
| 90 | 5 | 707 | 1713 | 75 |
| 95 | 5 | 881 | 2159 | 93 |
| 99 | 5 | 947 | 2328 | 99.69 |
| 100 | 5 | 950 | 2336 | 100 |
| 90 | 30 | 912 | 262 | 96 |
| 95 | 30 | 940 | 275 | 98 |
| 99 | 30 | 949.6 | 278 | 99.96 |
| 100 | 30 | 950 | 278 | 100 |

### Beispiel 3:

Es wurde untersucht, ob organische R.O.Membranen für die Entfernung eines Wasser/Methanol-Gemisches bei gleichzeitig hoher Retention von Substrat und Produkt geeignet sind:
In einer Versuchsanlage gemäß Figur 1 wurde eine Testlösung aus 7 Gew.% Na-Tiglat, 5 Gew.% Wasser und 88 Gew.% Methanol bei 25°C und einem Druckunterschied von 30 bar an der Membran mittels einer Pumpe aus dem Reaktor über die Membraneinheit (R.O.Membran, Typ NTR 759) gepumpt und die Retention, sowie der Fluss bestimmt.
Zuvor wurde die Membran über 5 Tage bei Raumtemperatur und Normaldruck zur Stabilisierung in 5 Gew% Wasser/Methanol gelagert.

Ergebnis: 96% Retention von Na-Tiglat, 2 kg/m²/h Fluss

### Beispiel 4:

Es wurde untersucht, ob organische R.O.Membranen unter Singlet Sauerstoff Oxidationsbedingungen stabil sind:
In einer Versuchsanlage gemäß Figur 1 wurde eine Testlösung aus 7 Gew.% Na-Tiglat, 5 Gew.% Wasser und 88 Gew.% Methanol bei 25°C und einem Druckunterschied von 30 bar an der Membran mittels einer Pumpe aus dem Reaktor über die Membraneinheit (R.O.Membran, Typ NTR 759) gepumpt und die Retention, sowie der Fluss bestimmt.
Zuvor wurde die Membran über 5 Tage Singlet Sauerstoff Oxidationsbedingungen ausgesetzt: 5 Gew% Wasser/Methanol, pH ca. 10, 0.02M Na₂MoO₄, H₂O₂-Zugabe bei einem H₂O₂/Katalysator-Molverhältnis von 3, Raumtemperatur, Normaldruck; die Lösung wurde 6 mal täglich erneuert.

Ergebnis: 85% Retention von Na-Tiglat, 13-16 kg/m²/h Fluss

### Beispiel 5:

Es wurde untersucht, ob eine keramische Pervaporationsmembran für die Entfernung eines Wasser/Methanol-Gemisches bei gleichzeitig hoher Retention von Substrat und Produkt geeignet ist:

In einer Versuchsanlage gemäß Figur 1 wurde eine Testlösung aus 7 Gew.% Na-Tiglat, 5 Gew.% Wasser und 88 Gew.% Methanol bei 25°C und einem Druckunterschied von 7 bar an der Membran mittels einer Pumpe aus dem Reaktor über die Membraneinheit (Sulzer Pervap SMS) gepumpt und die Retention, sowie der Fluss bestimmt.

Ergebnis: 100% Retention von Na-Tiglat, 0.1 kg/m²/h Fluss; Selektivität Wasser/Methanol = 6

## Patentansprüche

1. Verbessertes Verfahren zur Oxidation von organischen Substraten mittels ¹O₂, **dadurch gekennzeichnet, dass** wasserlösliche, oder in mit Wasser mischbaren organischen Lösungsmitteln lösliche, organische Substrate, die mit ¹O₂ reagieren, in einem mit Wasser mischbaren organischen Lösungsmittel, in Wasser oder in einem Gemisch aus Wasser und mit Wasser mischbaren organischem Lösungsmittel in Gegenwart eines heterogenen oder homogenen Katalysators mit 3-90%igem H₂O₂ versetzt werden, worauf anschließend an die katalytische Zersetzung von H₂O₂ zu Wasser und ¹O₂ die Oxidation der Substrate zu den entsprechenden Oxidationsprodukten erfolgt, wobei während der Reaktion Wasser selektiv mittels Membranen aus dem Reaktionsgemisch entfernt wird.

2. Verbessertes Verfahren nach Anspruch 1, **dadurch gekennzeichnet, dass** als Substrate, die mit ¹O₂ reagieren, Olefine, die 1 bis 10 C=C-Doppelbindungen enthalten; C₆-C₃₀ Aromaten; polycyclische Aromaten mit 2 bis 8 aromatischen Ringen; Alkylsulfide, Alkenylsulfide, Arylsulfide, die am Schwefelatom entweder monooder disubstituiert sind, sowie C₄-C₃₀ Heterocyclen mit einem oder mehreren O-, N- oder S-Atomen im Ring eingesetzt werden, die unsubstituiert sein können oder ein- oder mehrfach mit Halogenen, Cyanid, Carbonylgruppen, Hydroxylgruppen, C₁-C₂₀ Alkoxygruppen, C₁-C₂₀ Alkylgruppen, C₆-C₃₀ Arylgruppen, C₂-C₂₀ Alkenylgruppen, C₂-C₂₀ Alkinylgruppen, Carbonsäuregruppen, Estergruppen, Amidgruppen, Aminogruppen, Nitrogruppen, Silylgruppen, Silyloxygruppen, Sulfongruppen, Sulfoxidgruppen oder mit einem oder mehreren NR¹ R²-Resten, in denen R₁ oder R₂ gleich oder verschieden sein können und H; C₁-C₂₀ Alkyl; Formyl; C₂-C₂₀ Acyl; C₇-C₃₀ Benzoyl bedeuten, wobei R¹ und R² auch gemeinsam einen Ring bilden können, substituiert sein können.

3. Verbessertes Verfahren nach Anspruch 1, **dadurch gekennzeichnet, dass** als organisches mit Wasser mischbares Lösungsmittel C₁-C₈-Alkohole, Ethylenglykol, Propylenglykol, Formamid, N-Methylformamid, Dimethylformamid, Sulfolan, Dioxan,THF oder 1,2-Dimethoxyethan eingesetzt wird.

4. Verbessertes Verfahren nach Anspruch 1, **dadurch gekennzeichnet, dass** Katalysatoren basierend auf Molybdän, Wolfram, Scandium, Vanadium, Titan, Zirkon, Praseodym, Neodym, Samarium, Europium, Terbium, Dysprosium, Holmium, Erbium, Ytterbium oder Lutetium in Form von Oxiden, Oxokomplexen, Nitraten, Carboxylaten, Hydroxiden, Layered Double Hydroxiden, Carbonaten, Chloriden, Fluoriden, Sulfaten oder Tetrafluorboraten eingesetzt werden.

5. Verbessertes Verfahren nach Anspruch 1, **dadurch gekennzeichnet, dass** in Abhängigkeit vom eingesetzten Substrat 2 bis 10 Äquivalente an H₂O₂ eingesetzt werden.

6. Verbessertes Verfahren nach Anspruch 1, **dadurch gekennzeichnet, dass** die Reaktionstemperatur zwischen 0 und 50°C liegt.

7. Verbessertes Verfahren nach Anspruch 1, **dadurch gekennzeichnet, dass** zur selektiven Wasserentfernung organische oder anorganische Membranen eingesetzt werden.

8. Verbessertes Verfahren nach Anspruch 7, **dadurch gekennzeichnet, dass** als organische Membranen für die umgekehrte Osmose auf Basis von Polyvinylalkohol, Polyamid oder Polysulfon oder Celluloseacetat, Cellulosediacetat, Cellulosetriacetat, Cellulose-diacetat/Cellulosetriacetat, Cellulosenitrat, Polypropylen, Polyimid, sulfonated polysulfones, Polyethersulfone, Polyacrylnitril, Polyimid/Polyetherimid, Polyvinylidenfluorid, Aramid oder Polypiperazin oder Gemische derselben oder Nanofiltrationsmembranen auf Basis von Polyvinylalkohol, Polypropylen, Polysulfon, Polyethersulfon, Polyacrylnitril, Polyimid/Polyetherimid, Polyvinylidenfluorid, Polyamid, Polypiperazin, Celluloseacetat, Cellulosenitrat, Aramid, Cellulosediacetat, Cellulosetriacetat oder Gemische derselben oder organische Pervaporationsmembranen auf Basis von Polydimethylsiloxane, Poly(1-trimethylsilyl-1-propin), Polyurethane, Polybutadien, Polyether-Block-Polyamide, Silicon-Polycarbonate, Styrol-Butadien-Kautschuk, Nitril-Butadien-Kautschuk, Ethen-Propen-Terpolymer, Polyvinylalkohol, Polyamid, Polysulfon, Celluloseacetat, Aramid, Cellulosediacetat, Cellulosetriacetat, Polypiperazin oder Gemische derselben eingesetzt werden.

9. Verbessertes Verfahren nach Anspruch 7, **dadurch gekennzeichnet, dass** keramische Pervaporations-, Zeolith- oder Nanofiltrationsmembranen eingesetzt werden.

10. Verbessertes Verfahren nach Anspruch 1, **dadurch gekennzeichnet, dass** die eingesetzten Membranen einen Retentionsfaktor für Substrat und Produkt von mindestens 85% aufweisen.

11. Verbessertes Verfahren nach Anspruch 1, **dadurch gekennzeichnet, dass** während der Reaktion Wasser, das gegebenenfalls als Lösungsmittel eingesetzt wird und Wasser, das durch die 3-90%ige H₂O₂-Lösung eingebracht und das bei der katalysierten Disproportionierung von H₂O₂ gebildet wird, selektiv aus dem Reaktionsgemisch entfernt wird, indem das Reaktionsgemisch aus einem Reaktor (1) über eine Pumpe (3) in die Membraneinheit (4) gepumpt wird, in der sodann das Wasser durch eine geeignete Membran abgetrennt wird, wobei der Katalysator, im Falle eines homogenen Katalysators, das noch nicht umgesetzte Substrat und bereits gebildetes Produkt zurückgehalten und sogleich wieder in den Reaktor (1) eingeleitet werden und in Abhängigkeit von der gewählten Membran, das gegebenenfalls verwendete mit Wasser mischbare organische Lösungsmittel entweder ebenfalls zurückgehalten, oder aber mit dem Wasser abgetrennt wird, worauf anschließend in der Destillationssäule (5) eine destillative Abtrennung des Wassers vom organischen Lösungsmittel erfolgt, das sodann wieder über die Leitung (6) in den Reaktor (1) eingebracht und das abgetrennte Wasser verworfen wird.

12. Verbessertes Verfahren nach Anspruch 11, **dadurch gekennzeichnet, dass** anstelle einer Membraneinheit (4) zwei Membraneinheiten verwendet werden, wobei das Permeat der ersten Einheit in die zweite Membraneinheit geleitet und das Retentat der ersten Membraneinheit wieder in den Reaktor rückgeführt wird und anschließend das Retentat der zweiten Einheit ebenfalls wieder in den Reaktor geleitet wird, während vom Permeat der zweiten Einheit gegebenenfalls das organische mit Wasser mischbare Lösungsmittel abdestilliert wird oder, wenn das Permeat nur Wasser enthält, dieses verworfen wird.
